# EUROPEAN PATENT SPECIFICATION

(11) **EP 3 975 867 B1**
(45) Date of publication and mention of the grant of the patent: **16.11.2022**
(21) Application number: 20730224.1
(22) Date of filing: 29.05.2020
(51) Int. Cl.: A61B 8/08, A61B 8/14, A61B 8/00

(54) **METHODS AND SYSTEMS FOR GUIDING THE ACQUISITION OF CRANIAL ULTRASOUND DATA**
VERFAHREN UND SYSTEME ZUR ANLEITUNG DER AUFNAHME VON ULTRASCHALLDATEN DES SCHÄDELS
PROCÉDÉS ET SYSTÈMES DE GUIDAGE DE L'ACQUISITION DE DONNÉES ULTRASONORES CRÂNIENNES

(30) Priority: 31.05.2019 US 201962855021 P
(43) Date of publication of application: 06.04.2022
(73) Proprietor: Koninklijke Philips N.V., 5656 AG Eindhoven (NL)
(72) Inventor: SUTTON, Jonathan, Thomas, 5656 AE Eindhoven (NL); RAJU, Balasundar, Iyyavu, 5656 AE Eindhoven (NL); BHARAT, Shyam, 5656 AE Eindhoven (NL); FINCKE, Jonathan, 5656 AE Eindhoven (NL); SETHURAMAN, Shriram, 5656 AE Eindhoven (NL); SRINIVASA NAIDU, Raghavendra, 5656 AE Eindhoven (NL)
(74) Representative: Philips Intellectual Property & Standards
(86) International application number: PCT/EP2020/064975
(87) International publication number: WO 2020/239979

(56) References cited:
- WO-A1-2013/152035
- US-A1- 2002 103 436
- US-A1- 2012 022 377
- US-A1- 2012 083 717

## Description

### FIELD OF THE INVENTION

The invention relates to the field of ultrasound imaging, and more specifically to the field of cranial ultrasound imaging.

### BACKGROUND OF THE INVENTION

Cerebrovascular hemodynamic measurements are used to diagnose and monitor many conditions in the adult and pediatric populations. Radio-opaque CT tracers and MRI contrast agent techniques typically provide poor temporal resolution to assess hemodynamics adequately, require extensive equipment and setup, and are expensive.

Transcranial Doppler (TCD) ultrasound techniques may be used to monitor hemodynamics at the point-of-care with excellent temporal resolution in a non-invasive manner and at a relatively low cost. TCD can detect and monitor intracranial aneurysms, patent foramen ovale, vasospasm, stenosis, brain death, shunts, and microemboli in a surgical or ambulatory setting without radiation. Further, accurate TCD measurements can enable non-invasive measurement of intracranial pressure (nICP), which is a key indicator of cerebrovascular status as a result of stroke, tumor growth, or due to head trauma. Other ICP monitoring methods are typically highly invasive, requiring surgical penetration of the skull to place intra-parenchymal or ventricular sensors and are thus restricted to severe cases where monitoring and/or cerebrospinal fluid (CSF) drainage is required. The management of TBI in the early minutes at the point of injury has been suggested to impact patient outcomes profoundly, leading to evidence-based prehospital and in-hospital TBI treatment guidelines as outlined in N. Badjatia et al., "Guidelines for Prehospital Management of Traumatic Brain Injury 2nd Edition," Prehosp. Emerg. Care, vol. 12, no. supl, pp. S1-S52, Jan. 2008.

Currently, consistent TCD measurements are difficult to obtain due to the attenuation and aberration of the skull bone and the variability and tortuosity of perforating cerebral vessels. As a result, TCD must be performed by users with a considerable level of specialized training using single element transducers as described in A. V. Alexandrov et al., "Practice Standards for Transcranial Doppler (TCD) Ultrasound. Part II. Clinical Indications and Expected Outcomes," J. Neuroimaging, vol. 22, no. 3, pp. 215-224, Jul. 2012. This need for experienced operators significantly limits the scope of TCD as a clinical tool. Further, experienced operators exhibit substantial inter-operator variability in their measurements.

If novice ultrasound users could operate the devices in a consistent manner, then TCD could be routinely performed in settings such as emergency rooms, rural medical centers, battlefields, and ambulances for continuous monitoring, triage, and evidence-based application of therapy for a plurality of conditions involving cerebrovasculature.

US 2012/022377 A1 discloses a transcranial Doppler probe including a spherical bearing, a piezoelectric transducer pivotally attached to the spherical bearing, and first and second rods coupled to the piezoelectric transducer. The first rod is configured to pivot the piezoelectric transducer around a first pivot axis and the second rod is configured to pivot the piezoelectric transducer around a second pivot axis.

US 2002/103436 A1 discloses a method and apparatus comprising an intelligent transcranial Doppler (i-TCD) probe assembly which includes a transcranial Doppler (TCD) transducer, bi-temporal probe hanger, cylindrical probe housing with inner wiring, probe cylindrical base with coil, probe roller balls, spring system, a removable handle and software program for adjusting the angulation of the TCD probe and a microprocessor operatively connected to the transcranial Doppler device.

There is therefore a need for a means of guiding the acquisition of cerebral ultrasound data.

### SUMMARY OF THE INVENTION

The invention is defined by the claims.

According to examples in accordance with an aspect of the invention, there is provided a computer-implemented method for guiding the acquisition of ultrasound data within a 3D field of view, the method comprising:
obtaining initial 2D B-mode ultrasound data of a cranial region of a subject from a reduced field of view at a first imaging location;
determining whether a vessel of interest is located within the 3D field of view based on the initial 2D B-mode ultrasound data from said first imaging location;
if the vessel of interest is not located within the 3D field of view:
   generating a guidance instruction based on the initial 2D B-mode ultrasound data, wherein the guidance instruction is adapted to indicate a second imaging location to obtain further ultrasound data; and
if the vessel of interest is located within the 3D field of view:
   obtaining 3D Doppler ultrasound data of the cranial region from the 3D field of view.

The method provides for the guided acquisition of the 3D color Doppler ultrasound data of a vessel of interest in a cranial region.

By locating the vessel of interest first by way of 2D B-mode ultrasound data using a restricted field of view, the frame rate of the ultrasound data may be greatly increased, thereby increasing the accuracy of the localization of the vessel of interest.

In an embodiment, determining whether a vessel of interest is located within the 3D field of view comprises:
identifying an anatomical feature, for example a bone structure, within the reduced field of view based on the initial 2D B-mode ultrasound data; and
determining a likelihood of the vessel being located within the 3D field of view based on the identified anatomical feature.

Typical vessels of interest in the cranial region are located in close proximity to other distinct anatomical features, such as bone structures. Thus, by identifying such structures within the ultrasound data, the location of the vessel of interest may be determined within a given likelihood. The likelihood of a vessel being located within the 3D field of view can be expressed in one of two ways: either determining that the vessel is present; or informing the user that the vessel is not present with the further instructions on the second imaging location, wherein the further instructions can be based on comparative analyses of images from different modalities.

In a further embodiment, determining the likelihood of the vessel being located within the 3D field of view based on the identified anatomical feature comprises obtaining 2D color Doppler ultrasound data from a reduced field of view at a first imaging location.

In this way, a quick Doppler image may be provided to confirm whether the vessel of interest is indeed within the field of view of the probe. The 2D color Doppler ultrasound may be acquired from the reduced field of view using the structural information obtained from the 2D B-mode image data in order to check that flow exists where expected.

In an embodiment, determining whether a vessel of interest is located within the 3D field of view comprises applying a convolutional neural network to the initial 2D B-mode ultrasound data.

In a further embodiment, the convolution neural network is trained using 2D duplex color Doppler data.

Duplex color Doppler data comprises 2D Doppler images overlaid onto B-mode images. Thus, the locations of the vessels (as shown by the Doppler images) may be shown in relation to the structural features (as shown by the B-mode data). By training the network using these images, the network may infer the presence of a vessel of interest based on B-mode data alone.

In an embodiment, the ultrasound data is obtained by way of an ultrasound probe and wherein the method further comprises:
determining an orientation of the ultrasonic probe at the first imaging position; and
generating a probe manipulation instruction based on the orientation of the ultrasonic probe and the initial 2D B-mode ultrasound data, wherein the probe manipulation instruction is adapted to indicate how the ultrasound probe should be adjusted to reach the second imaging position.

In this way, the user or an automated system, may be instructed how to manipulate the probe in order to achieve an optimal view of the vessel of interest.

In a further embodiment, determining the orientation of the ultrasound probe comprises:
obtaining tracking data relating to the orientation of the probe; and
applying a second convolutional neural network to the tracking data.

The neural network may be trained to recognize the difference between a current orientation and previous, correct orientation, and generate the guidance accordingly.

In an embodiment, if the vessel of interest is located within the 3D field of view, the method further comprises:
measuring a bone structure within the 3D field of view;
generating a kernel for spatial filtering of the bone structure; and
applying the kernel to the 3D Doppler ultrasound data.

In this way, interference from structures within the field of view, but not of interest, may be reduced, or removed.

In an embodiment, the method further comprises:
periodically obtaining additional 2D B-mode ultrasound data;
comparing the additional 2D B-mode ultrasound data to the initial 2D B-mode ultrasound data; and
determining a movement of the vessel of interest based on the comparison.

In this way, over the movement of the vessel of interest over time (for example due to subject movement) may be monitored and corrected for.

In a further embodiment, the ultrasound data is obtained by way of an ultrasound probe and wherein the method further comprises determining a movement of the ultrasound probe based on the comparison.

In this way, over the movement of the probe of interest over time (for example due to user movement) may be monitored and corrected for.

According to examples in accordance with an aspect of the invention, there is provided a medical system adapted to guide the acquisition of ultrasound data within a 3D field of view, the system comprising:
a processor, wherein the processor is adapted to:
obtain initial 2D B-mode ultrasound data of a cranial region of a subject from a reduced field of view at a first imaging location;
determine whether a vessel of interest is located within the 3D field of view based on the initial 2D B-mode ultrasound data from said first imaging location;
if the vessel of interest is not located within the 3D field of view:
   generate a guidance instruction based on the initial 2D B-mode ultrasound data, wherein the guidance instruction is adapted to indicate a second imaging location to obtain further ultrasound data; and
if the vessel of interest is located within the 3D field of view:
   obtain 3D Doppler ultrasound data of the cranial region from the 3D field of view.

In an embodiment, the system further comprises an ultrasound probe in communication with the processor, wherein the ultrasound transducer is adapted to acquire 2D ultrasound data and 3D ultrasound data, and wherein the processor is adapted to initiate the ultrasound probe in a 2D B-mode ultrasound acquisition mode with a restricted field of view, and, if the vessel of interest is located within the full 3D field of view, switch the ultrasound probe to a 3D color Doppler ultrasound acquisition mode with a full field of view.

In a further embodiment, the system further comprises a probe tracker adapted to generate tracking data relating to the orientation of the ultrasound probe and wherein the processor is further adapted to determine the orientation of the probe based on the tracking data.

In a further embodiment, the probe track comprises one or more of:
an optical tracker; and
a motion tracker.

In an embodiment, the system further comprises a probe holder adapted to receive the ultrasound probe and hold the ultrasound probe in a given imaging position, wherein the probe holder is adapted to selectively lock the ultrasound probe in the given imaging position.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows an ultrasound diagnostic imaging system to explain the general operation;
Figure 2 shows a method of the invention;
Figures 3A and 3B show examples of the spatial relationship between vessels of interest and structures within the skull of a subject.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for guiding the acquisition of ultrasound data within a 3D field of view. The method begins by obtaining initial 2D B-mode ultrasound data of a cranial region of a subject from a reduced field of view, compared to the 3D field of view, at a first imaging location and determining whether a vessel of interest is located within the 3D field of view based on the initial 2D B-mode ultrasound data acquired within said reduced field of view. If the vessel of interest is not located within the 3D field of view, a guidance instruction is generated based on the initial 2D B-mode ultrasound data, wherein the guidance instruction is adapted to indicate a second imaging location to obtain further ultrasound data. If the vessel of interest is located within the 3D field of view, the 3D Doppler ultrasound data is obtained of the cranial region from the 3D field of view.

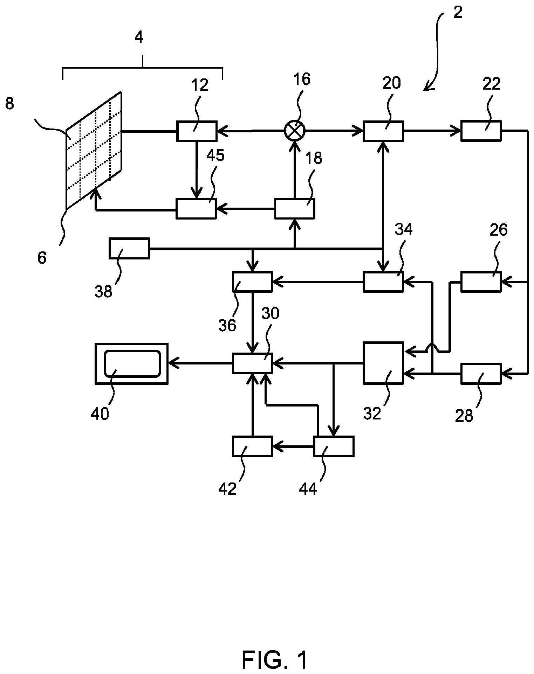

The general operation of an exemplary ultrasound system will first be described, with reference to Figure 1, and with emphasis on the signal processing function of the system since this invention relates to the processing of the signals measured by the transducer array.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. Therefore, the controller 18 enables a possibility to direct beam steering within the reduced field of views in a 3D volume corresponding to the 3D field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Figure 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals (representative of the acquired ultrasound data) may then be coupled to a B-mode (i.e. brightness mode, or 2D imaging mode) processor 26 and/or a Doppler processor 28. The B-mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and/or Doppler processors can be coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B-mode structural image with colors corresponding to motion at points in the image field, where the Doppler-estimated velocities to produce a given color. The combined B-mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D ultrasound images are coupled from the scan converter 32, multi-planar reformatter 44, and volume renderer 42 to an image processor 30 for further analyses, enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B-mode processor 26 can be coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor can be coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

The methods described herein may be performed on a processing unit. Such a processing unit may be located within an ultrasound system, such as the system described above with reference to Figure 1. For example, the image processor 30 described above may perform some, or all, of the method steps detailed below. Alternatively, the processing unit may be located in any suitable system, such as a monitoring system, that is adapted to receive an input relating to a subject.

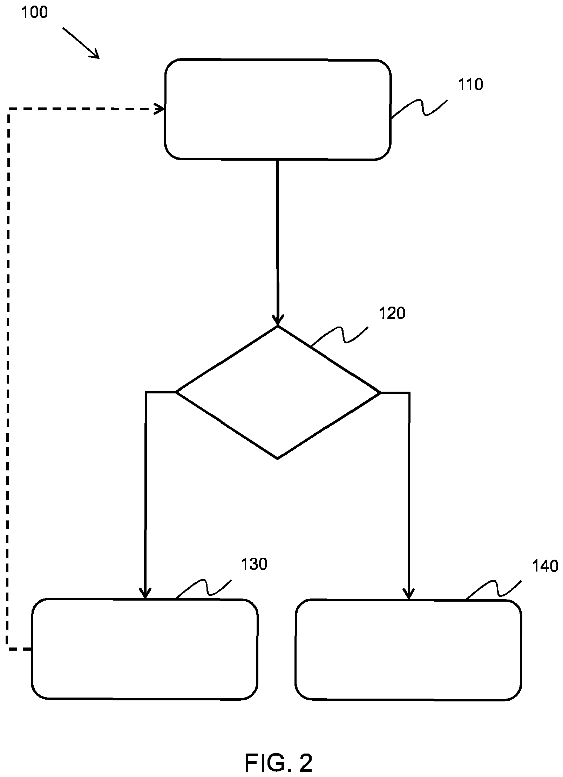

Figure 2 shows a computer-implemented method 100 for guiding the acquisition of ultrasound data within a 3D field of view.

The method begins in step 110 by obtaining initial 2D B-mode ultrasound data of a cranial region of a subject from a reduced field of view at a first imaging location, wherein said imaging location is within the 3D field of view.

The proposed method may be implemented as an image guidance routine that relies on the initial 2D B-mode ultrasound data to initially position an ultrasound probe, before switching to 3D color-Doppler, or power-Doppler, imaging routines to localize the vessel of interest and quantify the blood flow. In this way, the high frame rates available in 2D B-mode imaging may be used to accurately position the probe, and the robust flow quantification, acquisition of which often takes longer time, of 3D Doppler techniques to allow a user to obtain accurate measurements of a vessel of interest.

Put another way, the method provides a means to rapidly determine the accuracy of the position of the ultrasound probe for transcranial vessel imaging using high frame rate 2D B-mode imaging at the positioning stage, rather than using full 3D Doppler imaging.

In other words, by using 2D B-mode imaging, it is possible to rapidly orient the ultrasonic probe for subsequent flow monitoring using 3D Doppler imaging.

In step 120, it is determined whether a vessel of interest is located within the 3D field of view based on the initial 2D B-mode ultrasound data from the first imaging location.

The determination of whether the vessel of interest is located within the 3D field of view from the first imaging location may be performed in a number of ways.

For example, determining whether the vessel of interest is located within the 3D field of view may comprise identifying an anatomical feature, by the processor 30 in an non-limiting example, for example a bone structure, within the reduced field of view based on the initial 2D B-mode ultrasound data and determining a likelihood of the vessel being located within the 3D field of view based on the identified anatomical feature. Such an anatomical feature identification can be performed by comparing the acquired B-mode ultrasound data of the 2D ultrasound image with a database of other image modalities depicting the bone and vessel structures of the skull. The processor 30 may have means to access such a database and being adapted to applying a convolutional neural network to the initial 2D B-mode ultrasound data. Alternatively the step of anatomical feature identification can be performed in a different from the processor 30 physical location (such as cloud).

Magnetic resonance angiography, CT-angiography, digital subtraction angiography, and nuclear imaging of the brain and head have provided 3D atlases of other image modalities depicting the human cerebrovasculature. As a result, the location of major cerebral vessels, such as the MCA, is known to be consistently positioned relative to bony structures in the skull.

In other words, the likelihood of a vessel of interest being located inside an ultrasound probe's field of view may be determined based on the recognition of bony structures within the field of view.

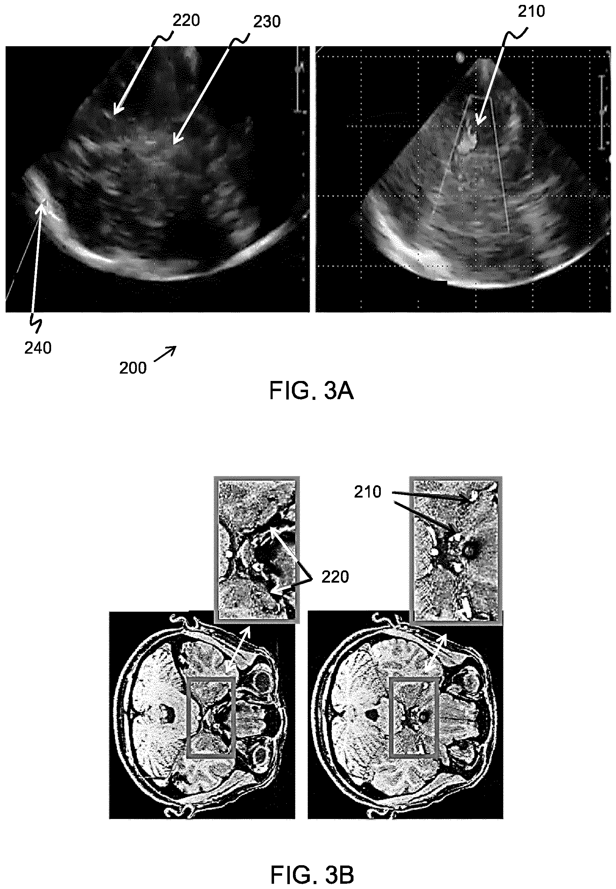

Examples of the locations of vessels of interest relative to structures within the skull are discussed below with reference to Figure 3A and 3B.

Determining the likelihood of the vessel being located within the 3D field of view based on the identified anatomical feature may include obtaining 2D color Doppler ultrasound data from a reduced field of view at a first imaging location.

The 2D color Doppler ultrasound data will reveal any movement, i.e. blood flow, within the reduced field of view. The direction of the flow relative to the imaging plane, as well as the size and rate of the flow, may be used to determine whether the vessel of interest is indeed being shown.

The determination of whether a vessel of interest is located within the 3D field of view may include applying the convolutional neural network to the initial 2D B-mode ultrasound data. For example, the convolutional neural network may be employed to recognize bony structures within the field of view, using any suitable image recognition technique, and determine a likelihood of a vessel occupying the field of view based on the recognized structures. The convolution neural network may be trained using 2D duplex color Doppler data, which is a combination of color Doppler data and B-mode data, meaning that the location of areas of flow (shown by the Doppler data) relative to the structures (shown by the B-mode data) may be learned.

If in step 120 it is determined that the vessel of interest is not located within the 3D field of view, the method progresses to step 130.

In step 130, generating a guidance instruction is generated based on the initial 2D B-mode ultrasound data, wherein the guidance instruction is adapted to indicate a second imaging location to obtain further ultrasound data. The method may then return to step 110 wherein the further ultrasound data is used instead of the initial ultrasound data.

The guidance instruction may vary based on the implementation of the method and system. For example, the ultrasound data may be obtained by way of an ultrasound probe. There are a number of implementations of an ultrasound probe that may be used to collect the ultrasound data as described above. For example, the ultrasound probe may comprise: a linear transducer array; a plurality of linear transducer arrays; or a 2D transducer array.

The guidance instruction provided to the user may be provided in an indirect manner. In other words, the system may analyze the initial ultrasound data and generate a guidance signal by way of a guidance means separate from the ultrasound probe. For example, where a visual guidance signal is generated, an arrow may be displayed on a screen (image display 40) indicating a direction in which the user should move the ultrasound probe. The screen can be also the same screen as the screen of the patient monitoring system.

Alternatively, the means for providing guidance to the user may be included in the ultrasound probe itself. For example, the ultrasound probe may be adapted to generate one or more of: an audible instruction; a visual instruction; an electronic control signal; and a tactile instruction, to guide the user to the second imaging position.

In the case of visual feedback, the ultrasound probe may be provided with one or more LEDs, which may provide a visual signal to a user as to how the ultrasound probe should be moved. In the case of an audible instruction, one or more speakers may be provided to supply an audible instruction to the user. Where tactile feedback is used, the ultrasound probe may be provided with one or more vibration modules, which (de)activate to provide a tactile instruction that may be interpreted by the user. In the example of an electronic instruction signal, the feedback may be provided to a remote digital display means, such as a monitor, which then presents the user with an instruction in a suitable form.

The guidance instruction may be generated using a convolution neural network, which is trained previous sets of initial ultrasound data and the movements required to arrive at the correct imaging position. In this way, the convolutional neural network may learn more efficient movements to bring the probe from the initial imaging position to the second imaging position.

It should be noted that any number of further imaging locations may be indicated by the user guidance information. For example, from the initial 2D B-mode ultrasound data, it may be determined that the vessel of interest may be imaged from several different imaging locations. In this case, a user guidance instruction may be generated for each of the alternative imaging locations and presented to the user, who may then select one of the alternative imaging locations to move the ultrasound probe to. Further, the system may store the alternative imaging locations for future user should further imaging be required.

If in step 120 it is determined that the vessel of interest is located within the 3D field of view, the method progresses to step 140.

In step 140, 3D Doppler ultrasound data is obtained from the cranial region using the 3D field of view. In other words, when the initial 2D B-mode ultrasound data has confirmed that the vessel of interest is within the 3D field of view, the imaging mode may be switched by the controller 18 which in also coupled to the processor 30, to the desired 3D Doppler imaging mode.

The 3D Doppler ultrasound data may then be used to analyze (by the Doppler processor 28, for example) the blood flow within the vessel of interest.

In addition, if the vessel of interest is determined to be located within the 3D field of view, the method may further include, using the processor 30 for example, measuring a bone structure within the 3D field of view, generating a kernel for spatial filtering of said bone structure and applying the kernel to the 3D Doppler ultrasound data.

In other words, structures within the field of view that may interrupt the 3D Doppler ultrasound data relating to the vessel of interest and cause interference in the final image may be filtered out using a specifically generated kernel.

The method described above may be carried out within an ultrasound system, such as the system described above with reference to Figure 1. Though separate functional units of the system were described and illustrated as separate elements in Figure 1, their exact implementation might be realized with the smaller amount of hardware elements, each performing several unit functions. However, this method may also be employed on any device that is capable of receiving ultrasound data. For example, an ultrasound probe may be used to acquire the ultrasound data, which may then be provided to a separate patient monitor to carry out the method described above. The data may be provided by any suitable communication means.

In the case that the ultrasound data is obtained directly by way of an ultrasound probe, the method may further include determining an orientation of the ultrasonic probe at the first imaging position and generating a probe manipulation instruction based on the orientation of the ultrasonic probe and the initial 2D B-mode ultrasound data.

The probe manipulation instruction may be adapted to indicate how the ultrasound probe should be adjusted to reach the second imaging position. The probe manipulation instruction may be delivered in a similar manner to the guidance instruction described above.

In addition, the method may also include periodically obtaining additional 2D B-mode ultrasound data, in particular, when in the 3D Doppler imaging mode. The additional 2D B-mode ultrasound data may be compared to the initial 2D B-mode ultrasound data in order to determine a movement of the vessel of interest within the field of view. Further, movement of an ultrasound probe may also be determined in this way.

Fresh guidance instructions may be generated based on the detected movement in order to ensure that the probe is held in the correct position when obtaining the 3D Doppler ultrasound data.

The method described above may make use of the fast 2D generalized Hough Transform as described in D. H. Ballard, "Generalizing the Hough transform to detect arbitrary shapes," Pattern Recognit., vol. 13, no. 2, pp. 111-122, Jan. 1981 and/or the template matching techniques described in Yuhai Li, Jian Liu, Jinwen Tian, and Hongbo Xu, "A fast rotated template matching based on point feature," 2005, vol. 6043, pp. 60431P-6043-7.

Figure 3A shows an example 200 of the spatial relationship between a cerebral vessel 210 and bone structures (such as the pterygopalatine fossa 220, which is adjacent to the vessel of interest in this example, the occipital base 230 and the frontal bone slope 240) within the head of a subject during an ultrasound examination. Here, the middle cerebral artery, the vessel of interest 210, is in close proximity to the frontal bone slope 240, occipital base 230 and pterygopalatine fossa 220, visible on a b-mode ultrasound image. Figure 3B shows the proximity of the pterygopalatine fossa 220 and vessels of interest in magnetic resonance (MR)-angiography.

By way of example, the method described above may be implemented as follows.

The user, for example a clinician, places an ultrasound probe over the temporal bone of the subject. In an example, the user may indicate via a user interface which vessel they wish to find. Guidance instructions may then be generated based on the user's choice.

The user may then coarsely manipulate the ultrasound probe, for example by performing millimeter translations and small angle articulations, until it is determined that the selected vessel of interest is within the field of view. At this stage, the ultrasound probe is operating in a 2D B-mode imaging mode, meaning that the probe guidance feedback to user should have a high update rate (e.g. > 10 Hz).

The user may be guided to fix the probe in place when it is determined that the probe is pointed towards the vessel. When an optimal probe location/orientation is identified, there may be an automatic transition of imaging system to 3D Doppler mode imaging and automatic placing of the initial position of the Doppler window based on expected location of vessel of interest.

As discussed above, the methods described may be implemented on a processor in any medical system. Looking to the example of an ultrasound system, the methods described above may be implemented in compact ultrasound system that is capable of operating in a combination of at least B-mode and color-Doppler imaging modes, and additionally power-Doppler and/or m-mode imaging modes. As the vessels of interest located in the cranial region of the subject are often situated in close proximity to echogenic bone structures, the ultrasound system may drive multiple plane acquisitions used for model training (for example, x-plane or multiple elevational planes).

The ultrasound system may include a transcranial ultrasound probe, which is a compact matrix ultrasound probe that can be manipulated by a user along the surface of the head anterior to the ear, enabling volumetric acquisitions of the cranial region of the subject.

If the vessel of interest is not found within the field of view of the probe, the user may be receive a guidance instruction relating to how the probe should be manipulated in order to achieve the desired view.

In an example, a convolutional neural network may be trained to provide probe guidance to the user in the form of a differential pose (i.e. translation and/or angulation required to position probe in an optimal orientation for vessel flow imaging). In this case, the initial 2D B-mode ultrasound data (or additionally duplex 2D color-, or power-, Doppler ultrasound data and/or multiplane 2D B-mode ultrasound data) is taken as an input to the network. The convolutional neural network may then output a differential pose (for example having 5 degrees of freedom of probe movement) required to orient the probe for imaging the vessel of interest.

This convolutional neural network may be trained in a number of ways. For example, ground truth data may be obtained in a prospective manner from a number of subjects. In an example, a series of subjects (e.g. 25 subjects) may undergo bilateral TCD scans using ultrasound probes having optical tracking markers rigidly fixed to the probe body and the patient's head in order to track the motion of the probe during the scanning process. TCD imaging of the vessel of interest would proceed according to normal standard-of-care by an experienced TCD sonographer, while tracking and duplex color-Doppler images are saved synchronously to memory. When the vessel of interest is located, data acquisition may be terminated. For each frame of ultrasound imaging, the differential pose relative to the pose in the final frame may be computed. In other words, for each 2D ultrasound image, the motion required to manipulate the probe to a location where the vessel of interest is centered within the probe's 3D field-of-view, is measured and used as a ground truth in the model training.

Once trained, the network may be used to generate a guidance instruction based on the incoming 2D B-mode ultrasound data that may be provided to the user for probe manipulation.

During the acquisition of the ultrasound data, the probe or the vessel may move. Accordingly, the system may be adapted to provide a rapid, flow-independent determination of probe or vessel motion.

For example, at periodic intervals, set by default and/or modified by the user, the system may compare a live 2D B-mode image to a set of pilot 2D B-mode images obtained after a successful localization of the vessel of interest. These images could be obtained in conventional 2D fashion, or in a multi-plane fashion (e.g. x-plane or multiple elevational planes). Image matching during probe reconfiguration may be performed using image cross correlation by comparing the live image iteratively to a set of elevational planes obtained after successful vessel of interest localization.

In addition, the probe may be provided with motion sensors. Tracking the motion of the probe during the probe manipulation phase can be combined with the 2D B-mode ultrasound data to determine coarse directional motion. Further, if it is determined that the vessel of interest has been lost from the field of view, a gyroscope recording of the period of motion can be used to guide the probe back toward its original orientation when the vessel of interest was localized.

The described methods and systems may be used to provide guidance to a user, or an automated system, that is manipulating an ultrasound probe. The ultrasound probe may be mounted in a device that selectively fixes the probe rigidly to the head of the subject. Such a device may accommodate one or more degrees of freedom of the probe and may be provided with a locking mechanism.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented method (100) for guiding the acquisition of ultrasound data within a 3D field of view, the method comprising:
obtaining (110) initial 2D B-mode ultrasound data of a cranial region of a subject from a reduced field of view at a first imaging location within the 3D field of view;
determining (120) whether a vessel of interest is located within the 3D field of view from said first imaging location based on the initial 2D B-mode ultrasound data; and
if the vessel of interest is not located within the 3D field of view:
generating (130) a guidance instruction based on the initial 2D B-mode ultrasound data, wherein the guidance instruction is adapted to indicate a second imaging location to obtain further ultrasound data, wherein the second imaging location is a different location within the 3D field of view from the first imaging location; and
if the vessel of interest is located within the 3D field of view:
obtaining (140) 3D Doppler ultrasound data of the cranial region from the 3D field of view.

2. The computer-implemented method as claimed in claim 1, wherein determining whether a vessel of interest is located within the 3D field of view comprises:
identifying an anatomical feature, for example a bone structure, within the reduced field of view based on the initial 2D B-mode ultrasound data; and
determining a likelihood of the vessel being located within the 3D field of view based on the identified anatomical feature.

3. The computer-implemented method as claimed in claim 2, wherein determining a likelihood of the vessel being located within the 3D field of view based on the identified anatomical feature comprises obtaining 2D color Doppler ultrasound data from a reduced field of view at a first imaging location.

4. The computer-implemented method as claimed in any of claims 1 to 3, wherein determining the likelihood of whether a vessel of interest is located within the 3D field of view comprises applying a convolutional neural network to the initial 2D B-mode ultrasound data.

5. The computer-implemented method as claimed in claim 4, wherein the convolution neural network is trained using 2D duplex color Doppler data.

6. The computer-implemented method as claimed in any of claims 1 to 5, wherein the ultrasound data is obtained by way of an ultrasound probe and wherein the method further comprises:
determining an orientation of the ultrasonic probe at the first imaging position; and
generating a probe manipulation instruction based on the orientation of the ultrasonic probe and the initial 2D B-mode ultrasound data, wherein the probe manipulation instruction is adapted to indicate how the ultrasound probe should be adjusted to reach the second imaging position.

7. The computer-implemented method as claimed in claim 6, wherein determining the orientation of the ultrasound probe comprises:
obtaining tracking data relating to the orientation of the probe; and
applying a second convolutional neural network to the tracking data.

8. The computer-implemented method as claimed in any of claims 1 to 7, wherein, if the vessel of interest is located within the 3D field of view, the method further comprises:
measuring a bone structure within the 3D field of view;
generating a kernel for spatial filtering of the bone structure; and
applying the kernel to the 3D Doppler ultrasound data.

9. The computer-implemented method as claimed in any of claims 1 to 8, wherein the method further comprises:
periodically obtaining additional 2D B-mode ultrasound data;
comparing the additional 2D B-mode ultrasound data to the initial 2D B-mode ultrasound data; and
determining a movement of the vessel of interest based on the comparison.

10. The computer-implemented method as claimed in claim 9, wherein the ultrasound data is obtained by way of an ultrasound probe and wherein the method further comprises determining a movement of the ultrasound probe based on the comparison.

11. A medical system adapted to guide the acquisition of ultrasound data within a 3D field of view, the system comprising:
a processor (30)
wherein the processor is adapted to:
obtain initial 2D B-mode ultrasound data of a cranial region of a subject from a reduced field of view at a first imaging location within the 3D field of view;
determine whether a vessel of interest is located within the 3D field of view based on the initial 2D B-mode ultrasound data from said first imaging location; and
if the vessel of interest is not located within the 3D field of view:
generate a guidance instruction based on the initial 2D B-mode ultrasound data, wherein the guidance instruction is adapted to indicate a second imaging location to obtain further ultrasound data, wherein the second imaging location is a different location within the 3D field of view from the first imaging location; and
if the vessel of interest is located within the 3D field of view:
obtain 3D Doppler ultrasound data of the cranial region from the 3D field of view.

12. The system as claimed in claim 11, wherein the system further comprises an ultrasound probe (4) in communication with the processor, wherein the ultrasound transducer is adapted to acquire 2D ultrasound data and 3D ultrasound data, and wherein the processor is adapted to initiate the ultrasound probe in a 2D B-mode ultrasound acquisition mode with a restricted field of view, and, if the vessel of interest is located within the full 3D field of view, switch the ultrasound probe to a 3D color Doppler ultrasound acquisition mode with a full field of view.

13. The system as claimed in claim 12, wherein the system further comprises a probe tracker adapted to generate tracking data relating to the orientation of the ultrasound probe and wherein the processor is further adapted to determine the orientation of the probe based on the tracking data.

14. The system as claimed in claim 13, wherein the probe track comprises one or more of:
an optical tracker; and
a motion tracker.

15. The system as claimed in any of claims 12 to 14, wherein the system further comprises a probe holder adapted to receive the ultrasound probe and hold the ultrasound probe in a given imaging position, wherein the probe holder is adapted to selectively lock the ultrasound probe in the given imaging position.

## Patentansprüche

1. Computer-implementiertes Verfahren (100) zum Leiten der Erfassung von Ultraschalldaten innerhalb eines 3D-Sichtfeldes, wobei das Verfahren Folgendes umfasst:
Erhalten (110) von anfänglichen 2D-B-Mode-Ultraschalldaten eines kranialen Bereichs eines Patienten aus einem reduzierten Sichtfeld an einem ersten Abbildungsort innerhalb des 3D-Sichtfelds;
Bestimmen (120), ob sich ein interessierendes Gefäß innerhalb des 3D-Sichtfeldes von der ersten Bildgebungsstelle aus befindet, basierend auf den anfänglichen 2D-B-Mode-Ultraschalldaten; und wenn sich das interessierende Gefäß nicht innerhalb des 3D-Sichtfeldes befindet:
Erzeugen (130) einer Führungsanweisung auf der Grundlage der anfänglichen 2D-B-Mode-Ultraschalldaten, wobei die Führungsanweisung geeignet ist, einen zweiten Abbildungsort anzugeben, um weitere Ultraschalldaten zu erhalten, wobei der zweite Abbildungsort ein anderer Ort innerhalb des 3D-Sichtfelds als der erste Abbildungsort ist; und
wenn sich das betreffende Gefäß innerhalb des 3D-Sichtfelds befindet:
Gewinnung (140) von 3D-Doppler-Ultraschalldaten der Schädelregion aus des 3D-Sichtfeldes.

2. Computerimplementiertes Verfahren nach Anspruch 1, wobei die Bestimmung, ob sich ein interessierendes Gefäß innerhalb des 3D-Sichtfelds befindet, Folgendes umfasst:
Identifizierung eines anatomischen Merkmals, z. B. einer Knochenstruktur, innerhalb des reduzierten Sichtfeldes auf der Grundlage der anfänglichen 2D-B-Mode-Ultraschalldaten; und
Bestimmung einer Wahrscheinlichkeit, dass sich das Gefäß innerhalb des 3D-Sichtfeldes befindet, auf der Grundlage des identifizierten anatomischen Merkmals.

3. Computerimplementiertes Verfahren nach Anspruch 2, wobei die Bestimmung der Wahrscheinlichkeit, dass sich das Gefäß innerhalb des 3D-Sichtfelds befindet, auf der Grundlage des identifizierten anatomischen Merkmals das Erhalten von 2D-Farbdoppler-Ultraschalldaten aus einem reduzierten Sichtfeld an einem ersten Abbildungsort umfasst.

4. Computerimplementiertes Verfahren nach einem der Ansprüche 1 bis 3, wobei die Bestimmung der Wahrscheinlichkeit, ob sich ein interessierendes Gefäß innerhalb des 3D-Sichtfelds befindet, die Anwendung eines neuronalen Faltungsnetzwerks auf die anfänglichen 2D-B-Mode-Ultraschalldaten umfasst.

5. Computerimplementiertes Verfahren nach Anspruch 4, wobei das neuronale Faltungsnetzwerk unter Verwendung von 2D-Duplex-Farbdopplerdaten trainiert wird.

6. Computerimplementiertes Verfahren nach einem der Ansprüche 1 bis 5, wobei die Ultraschalldaten mittels einer Ultraschallsonde gewonnen werden und wobei das Verfahren des Weiteren umfasst:
Bestimmung einer Ausrichtung der Ultraschallsonde an der ersten Abbildungsposition;
und
Erzeugen einer Sondenmanipulationsanweisung auf der Grundlage der Ausrichtung der Ultraschallsonde und der anfänglichen 2D-B-Mode-Ultraschalldaten, wobei die Sondenmanipulationsanweisung so angepasst ist, dass sie angibt, wie die Ultraschallsonde eingestellt werden sollte, um die zweite Abbildungsposition zu erreichen.

7. Computerimplementiertes Verfahren nach Anspruch 6, wobei die Bestimmung der Ausrichtung der Ultraschallsonde Folgendes umfasst:
Erhalten von Verfolgungsdaten, die sich auf die Ausrichtung der Sonde beziehen; und
Anwendung eines zweiten neuronalen Faltungsnetzwerks auf die Verfolgungsdaten.

8. Das computerimplementierte Verfahren nach einem der Ansprüche 1 bis 7,
wobei, wenn sich das interessierende Gefäß innerhalb des 3D-Sichtfeldes befindet, das Verfahren des Weiteren Folgendes umfasst:
Messung einer Knochenstruktur innerhalb des 3D-Sichtfelds;
Erzeugung eines Kernels für die räumliche Filterung der Knochenstruktur; und
Anwendung des Kernels auf die 3D-Doppler-Ultraschalldaten.

9. Das computerimplementierte Verfahren nach einem der Ansprüche 1 bis 8, wobei das Verfahren des Weiteren Folgendes umfasst:
regelmäßige Gewinnung zusätzlicher 2D-B-Mode-Ultraschalldaten;
Vergleichen der zusätzlichen 2D-B-Mode-Ultraschalldaten mit den ursprünglichen 2D-B-Mode-Ultraschalldaten; und
Bestimmung einer Bewegung des Gefäßes von Interesse auf der Grundlage des Vergleichs.

10. Computerimplementiertes Verfahren nach Anspruch 9, wobei die
die Ultraschalldaten mittels einer Ultraschallsonde gewonnen werden und wobei das Verfahren des Weiteren die Bestimmung einer Bewegung der Ultraschallsonde auf der Grundlage des Vergleichs umfasst.

11. Ein medizinisches System, das geeignet ist, die Erfassung von Ultraschalldaten innerhalb eines 3D-Sichtfeldes zu leiten, wobei das System Folgendes umfasst:
einen Prozessor (30), wobei der Prozessor dazu geeignet ist:
erste 2D-B-Mode-Ultraschalldaten eines Schädelbereichs einer Person aus einem reduzierten Sichtfeld an einer ersten Bildgebungsstelle innerhalb des 3D-Sichtfelds erhalten;
zu bestimmen, ob sich ein interessierendes Gefäß innerhalb des 3D-Sichtfeldes befindet, basierend auf den anfänglichen 2D-B-Mode-Ultraschalldaten von der ersten Abbildungsstelle; und
wenn sich das betreffende Gefäß nicht innerhalb des 3D-Sichtfelds befindet:
eine Führungsanweisung auf der Grundlage der anfänglichen 2D-B-Mode-Ultraschalldaten zu erzeugen, wobei die Führungsanweisung geeignet ist, einen zweiten Abbildungsort anzugeben, um weitere Ultraschalldaten zu erhalten, wobei der zweite Abbildungsort ein anderer Ort innerhalb des 3D-Sichtfelds als der erste Abbildungsort ist; und
wenn sich das betreffende Gefäß innerhalb des 3D-Sichtfelds befindet:
3D-Doppler-Ultraschalldaten der Schädelregion aus dem 3D-Sichtfeld zu erhalten.

12. System nach Anspruch 11, wobei das System des Weiteren eine Ultraschallsonde (4) in Kommunikation mit dem Prozessor umfasst, wobei der Ultraschallwandler so ausgelegt ist, dass er 2D-Ultraschalldaten und 3D-Ultraschalldaten erfasst, und wobei der Prozessor so ausgelegt ist, dass er die Ultraschallsonde in einem 2D-B-Mode-Ultraschall-Erfassungsmodus mit einem eingeschränkten Sichtfeld initiiert und, wenn sich das interessierende Gefäß innerhalb des vollständigen 3D-Sichtfeldes befindet, die Ultraschallsonde in einen 3D-Farbdoppler-Ultraschall-Erfassungsmodus mit einem vollständigen Sichtfeld umschaltet.

13. System nach Anspruch 12, wobei das System des Weiteren eine Sondenverfolgung umfasst, die geeignet ist, Verfolgungsdaten bezüglich der Ausrichtung der Ultraschallsonde zu erzeugen, und wobei der Prozessor des Weiteren geeignet ist, die Ausrichtung der Sonde auf der Grundlage der Verfolgungsdaten zu bestimmen.

14. System nach Anspruch 13, wobei die Sondenspur eines oder mehrere der folgenden Elemente umfasst:
einen optischen Peilsender; und
einen Bewegungsmelder.

15. System nach einem der Ansprüche 12 bis 14, wobei das System des Weiteren eine Sondenhalterung umfasst, die so angepasst ist, dass sie die Ultraschallsonde aufnimmt und die Ultraschallsonde in einer gegebenen Abbildungsposition hält, wobei die Sondenhalterung so angepasst ist, dass sie die Ultraschallsonde in der gegebenen Abbildungsposition selektiv arretiert.

## Revendications

1. Procédé mis en œuvre par ordinateur (100) pour guider l'acquisition de données ultrasonores dans un champ de vision 3D, le procédé comprenant:
l'obtention (110) de données ultrasonores initiales en mode B 2D d'une région crânienne d'un sujet à partir d'un champ de vision réduit à un premier emplacement d'imagerie dans le champ de vision 3D;
déterminer (120) si un vaisseau d'intérêt est situé dans le champ de vision 3D à partir dudit premier emplacement d'imagerie sur la base des données ultrasonores initiales en mode B 2D; et si le vaisseau d'intérêt n'est pas situé dans le champ de vision 3D:
générer (130) une instruction de guidage sur la base des données échographiques initiales en mode B 2D, dans laquelle l'instruction de guidage est adaptée pour indiquer un second emplacement d'imagerie pour obtenir des données ultrasonores supplémentaires, dans laquelle le deuxième emplacement d'imagerie est un emplacement différent à l'intérieur du champ de vision 3D par rapport au premier emplacement d'imagerie; et
si le vaisseau d'intérêt est situé dans le champ de vision 3D:
obtenir (140) des données ultrasonores Doppler 3D de la région crânienne à partir du champ de vision 3D.

2. Procédé mis en œuvre par ordinateur selon la revendication 1, dans lequel déterminer si un vaisseau d'intérêt est situé dans le champ de vision 3D comprend:
l'identification d'une caractéristique anatomique, par exemple une structure osseuse, dans le champ de vision réduit sur la base des données ultrasonores initiales en mode B 2D; et
déterminer une probabilité que le vaisseau soit situé dans le champ de vision 3D sur la base de la caractéristique anatomique identifiée.

3. Procédé mis en œuvre par ordinateur selon la revendication 2, dans lequel la détermination d'une probabilité que le vaisseau soit situé dans le champ de vision 3D sur la base de la caractéristique anatomique identifiée comprend l'obtention de données ultrasonores Doppler couleur 2D à partir d'un champ de vision réduit à un premier emplacement d'imagerie.

4. Procédé mis en œuvre par ordinateur selon l'une quelconque des revendications 1 à 3, dans lequel la détermination de la probabilité qu'un vaisseau d'intérêt soit situé dans le champ de vision 3D comprend l'application d'un réseau neuronal convolutif aux données ultrasonores initiales en mode B en 2D.

5. Procédé mis en œuvre par ordinateur selon la revendication 4, dans lequel le réseau neuronal convolutif est formé en utilisant des données Doppler couleur duplex 2D.

6. Procédé mis en œuvre par ordinateur selon l'une quelconque des revendications 1 à 5, dans lequel les données ultrasonores sont obtenues au moyen d'une sonde à ultrasons et dans lequel le procédé comprend en outre:
déterminer une orientation de la sonde à ultrasons à la première position d'imagerie; et
la génération d'une instruction de manipulation de la sonde basée sur l'orientation de la sonde à ultrasons et les données ultrasonores initiales en mode B 2D, dans laquelle l'instruction de manipulation de la sonde est adaptée pour indiquer comment la sonde à ultrasons doit être ajustée pour atteindre la deuxième position d'imagerie.

7. Procédé mis en œuvre par ordinateur selon la revendication 6, dans lequel la détermination de l'orientation de la sonde à ultrasons comprend:
l'obtention de données de suivi relatives à l'orientation de la sonde; et
l'application d'un deuxième réseau neuronal convolutif aux données de suivi.

8. Procédé mis en œuvre par ordinateur selon l'une quelconque des revendications 1 à 7,
dans lequel, si le vaisseau d'intérêt est situé à l'intérieur du champ de vision 3D, le procédé comprend en outre:
la mesure d'une structure osseuse dans le champ de vision 3D;
générer un noyau pour le filtrage spatial de la structure osseuse; et
l'application du noyau aux données ultrasonores Doppler 3D.

9. Procédé mis en œuvre par ordinateur selon l'une quelconque des revendications 1 à 8, dans lequel le procédé comprend en outre:
l'obtention périodique de données ultrasonores supplémentaires en mode B 2D;
comparer les données ultrasonores supplémentaires en mode B 2D aux données ultrasonores initiales en mode B 2D; et
déterminer un mouvement du vaisseau d'intérêt sur la base de la comparaison.

10. Méthode mise en œuvre par ordinateur selon la revendication 9, dans laquelle les données ultrasonores sont obtenues au moyen d'une sonde à ultrasons et dans lequel le procédé comprend en outre la détermination d'un mouvement de la sonde à ultrasonos sur la base de la comparaison.

11. Système médical adapté pour guider l'acquisition de données ultrasonores dans un champ de vision 3D, le système comprenant:
un processeur (30) dans lequel le processeur est adapté pour:
obtenir des données ultrasonores initiales en mode B 2D d'une région crânienne d'un sujet à partir d'un champ de vision réduit à un premier emplacement d'imagerie dans le champ de vision 3D;
déterminer si un vaisseau intéressant est situé dans le champ de vision 3D sur la base des données ultrasonores initiales en mode B 2D provenant dudit premier emplacement d'imagerie; et
si le vaisseau concerné n'est pas situé dans le champ d'observation 3D:
générer une instruction de guidage sur la base des données ultrasonores initiales en mode B 2D, dans laquelle l'instruction de guidage est adaptée pour indiquer un deuxième emplacement d'imagerie pour obtenir d'autres données ultrasonores, dans laquelle le deuxième emplacement d'imagerie est un emplacement différent à l'intérieur du champ d'observation 3D par rapport au premier emplacement d'imagerie; et
si le vaisseau d'intérêt est situé dans le champ de vision 3D:
obtenir des données ultrasonores Doppler 3D de la région crânienne à partir du champ de vision 3D.

12. Système selon la revendication 11, dans lequel le système comprend en outre une sonde à ultrasons (4) en communication avec le processeur, dans lequel le transducteur à ultrasons est adapté pour acquérir des données ultrasonores en 2D et des données ultrasonores en 3D, et dans lequel le processeur est adapté pour lancer la sonde à ultrasons dans un mode d'acquisition à ultrasons en mode B en 2D avec un champ de vision restreint, et, si le vaisseau concerné est situé dans le champ de vision complet en 3D, commuter la sonde à ultrasons vers un mode d'acquisition à ultrasons Doppler couleur en 3D avec un champ de vision complet.

13. Système selon la revendication 12, dans lequel le système comprend en outre un dispositif de suivi de sonde adapté pour générer des données de suivi concernant l'orientation de la sonde à ultrasons et dans lequel le processeur est en outre adapté pour déterminer l'orientation de la sonde sur la base des données de suivi.

14. Système selon la revendication 13, dans lequel la piste de la sonde comprend une ou plusieurs des caractéristiques suivantes:
un suiveur optique; et
un suiveur de mouvement.

15. Système selon l'une quelconque des revendications 12 à 14, dans lequel le système comprend en outre un support de sonde adapté pour recevoir la sonde à ultrasons et maintenir la sonde à ultrasons dans une position d'imagerie donnée, dans lequel le support de sonde est adapté pour verrouiller sélectivement la sonde à ultrasons dans la position d'imagerie donnée.
